# EUROPEAN PATENT APPLICATION

(11) **EP 0 917 880 A1**
(43) Date of publication of application: **26.05.1999**
(21) Application number: 97116710.1
(22) Date of filing: 25.09.1997
(51) Int. Cl.: A61K 47/40

(54) **Inclusion complex of diiodomethyl-p-tolylsulfone with cyclodextrin derivatives**

(71) Applicant: Wacker-Chemie GmbH, 81737 München (DE)
(72) Inventor: Wimmer, Thomas, Dr., 84533 Marktl (DE); Saitoh, Akihisa, Dr., Yamashina-Ku, Kyoto, 607-8177 (JP)
(74) Representative: Potten, Holger

(57) **Abstract**

The invention relates to an inclusion complex of Amical (diiodomethyl-p-tolylsulfone) with cyclodextrin derivatives and the use of this inclusion complex for the preservation of products.

## Description

The invention relates to an inclusion complex of Amical (diiodomethyl-p-tolylsulfone) with cyclodextrin derivatives and its use.

Cyclodextrins are cyclic oligosaccharides made enzymatically from starch. Commercially available cyclodextrins are α-, β-or γ-cyclodextrins consisting of 6, 7 or 8 anhydro glucose units.

Cyclodextrins can be chemically modified, for example, by etherfication or esterfication to obtain cyclodextrin derivatives. The production of such cylodextrin derivatives is state of the art. Different methylated or hydroxypropylated cyclodextrins are available commercially from Wacker-Chemie GmbH, Munich.

Alkylated or acylated cyclodextrins are characterized by the average degree of substitution (DS), which is the average number of substituted hydroxy functions per glucose unit. Because in case of hydroxyalkylation the substituents can be further hydroxyalkylated those cyclodextrin derivatives are characterized by the term average molar substitution (MS) which is the average number of moles of the substituting agent per glucose unit.

Through their cavity cyclodextrins and cyclodextrin derivatives are capable to form inclusion complexes with a great variety of organic molecules.

Microbial activity within products containing an aqueous phase may lead to discoloration, decomposition, change of texture and/or development of bad odor of the product. By the use of biocides these problems can be solved.

Inclusion complexes of biocides with cyclodextrins are already known from literature.

CA 100: 156595 discloses a β-cyclodextrin complex of 2-N-octyl-4-isothiazolin-3-one having a low skin irritation.

CA 109: 134972 describes a water soluble iodine complex with maltosyl cyclodextrins which can be used in mouthwashes.

CA 119: 175868 discloses inclusion complexes of several water soluble biocides with β-cyclodextrin.

CA 120:71552 discloses the complexation of isothiazolinones with β-cyclodextrin. A reduced skin irritation was found for the 4,5-dichloro-2-N-octyl-4-isothiazolin-3-one inclusion complex with β-cyclodextrin.

An increase of the water solubility of p-hydroxybenzoic acid esters (parabens) by complexation with hydroxypropyl-β-cyclodextrin is described in CA 120:62177. A reduced antimicrobial activity of the product was found.

DE 4313408 discloses an increase of the water solubility of biocides having a solubility lower than 0.15 g/l by inclusion into cyclodextrins. Also the use of such compounds for the preservation of aqueous systems is described.

US 5403828 discloses the purification of cyclodextrin complexes. As Example for the purification of a cyclodextrin derivative the purification of a β-cyclodextrin complex with Amical is disclosed in Example 1. The complex is not characterized in the patent.

Amical is known as a preservation agent with fungicidal activity. A great disadvantage of Amical is its extremely low watersolubility of less than 5 ppm.

It is an object of the present invention to provide formulations of Amical which have an enhanced water solubility and an increased activity against microorganisms in comparison to pure amical.

The present invention is related to an inclusion complex of Amical with a cyclodextrin derivative.

The inclusion complex according to the invention increases the solubility of Amical in water. It further has a higher biological efficacy than pure amical.

The inclusion complex according to the invention contains as cyclodextrin derivative any known cyclodextrin derivative.

Preferred as cyclodextrin derivative in the inclusion complex according to the invention are alkylated or hydroxyalkylated cyclodextrin derivatives.

Particularly preferred as cyclodextrin derivative in the inclusion complex according to the invention are methylated cyclodextrin derivatives, such as, for example, methyl-α-cyclodextrin, methyl-β-cyclodextrin or methyl-γ-cyclodextrin or mixtures thereof.

Especially preferred as cyclodextrin derivative in the inclusion complex according to the invention is methyl-β-cyclodextrin.

The cyclodextrin derivatives have preferable a degree of substitution of 0.3 - 2.6.

Particularly preferred is a degree of substitution of 0.5 - 2.0.

The inclusion complex according to the invention contains preferably cyclodextrin derivative and amical in a ratio of 1 : 0.05 to 1 : 1 (molar).

A further object of the present invention is a process for the production of an inclusion complex according to the present invention.

This process is characterized by stirring, shaking, or kneading of Amical with a cyclodextrin derivative in an appropriate solvent and drying the resulting product.

As solvent may preferably be used at least one compound selected from the group water, C1 - C4 alcohols, acetone, methyl ethyl ketone, ethyl acetate, methyl acetate, dimethyl sulfoxide, dimethyl formamide and pyridine.

Preferred as a solvent is water, ethanol or a mixture thereof.

Aqueous formulations can be dried, for example by spray-drying or freeze-drying, to a solid inclusion complex according to the invention.

Solid complexes can be ground or formed into granules or tablets.

A further object of the present invention is an aqueous solutions of the inclusion complex according to the invention.

This solution contains besides the inclusion complex according to the invention preferably at least one compound selected from, the group C1 - C4 alcohols, acetone, methyl ethyl ketone, ethyl acetate, methyl acetate, dimethyl sulfoxide, dimethyl formamide and pyridene.

The solution according to the invention may further contain at least one compound selected from, the group anti-foaming agent, corrosion inhibitor, emulsifier, inorganic water-soluble salt and surfactant.

If water is used as a solvent, clear, concentrate, water dilutable solutions are obtained.

Preferably the inclusion complex according to the invention is present in 0.5 % to 60 % (w/w) in the aqueous solution.

The solution according to the invention can be made by omitting the drying step in the process for the production of the inclusion complex according to the present invention or by dissolving the inclusion complex in the respective solvent.

A further object of the invention is the use of the inclusion complex according to the present invention for the preservation of products.

For this purpose the complex according to the present invention or an aqueous solution thereof is added to water containing products to prevent or diminish the growth of microorganism.

Surprisingly the biological efficacy of the inclusion complex according to the present invention is by the factor 10 to 40 higher than the biological efficacy of not complexed Amical.

The inclusion compounds of the present invention can be used for the preservation of paints, lacquers, aqueous emulsion and dispersion, metal working fluids, cosmetic products, cleansing agents. For this purpose the inclusion complex or the aqueous solution is added to the respective product in an amount of 2 mg/l to 500 mg/l, preferably 2 mg/l to 100mg/l.

A further object of the present invention are paints, lacquers, aqueous emulsion and dispersion, metal working fluids, cosmetic products, cleansing agents characterized in that they contain an inclusion complex according to the present invention.

The following examples serve to further explain the invention.

### Example 1: Phase solubility isotherms with different cyclodextrin derivatives

An amount corresponding to 10.0 g of dry cyclodextrin derivative was weight in a 100 ml volumetric flask, dissolved and filled up to the mark by 0.1 molar aqueous phosphate buffer (K₂HPO₄ / KH₂PO₄) solution. Cyclodextrin derivative solutions from 1% to 10% were prepared by dilution from this 10% (w/v) cyclodextrin stock solution. As comparison example a 0.1 molar phosphate buffer was used.
20 mg of Amical were added to 1 ml of each solution and the mixtures were shaken for 72 hours.

The samples were filtrated through a 0.2 µm filter. The Amical concentration of the clear solutions were measured by HPLC (Column: Nucleosil C18, 7 µm, 25 cm; mobile phase: 56 vol. % ethanol, 44 vol. % water, 2 vol. % acetonitrile, detection UV at λ = 250 nm)

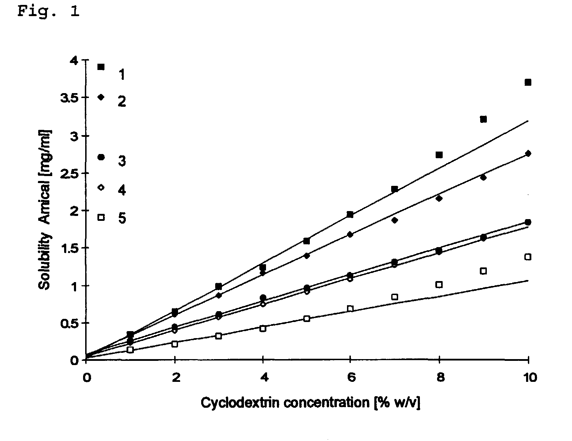

Fig. 1 shows the interdependence of the amical concentration and the concentration of the cyclodextrin derivatives in the solutions.

In Fig. 1
1 means Methyl-β-cyclodextrin (DS=1.8)
2 means Methyl-β-cyclodextrin (DS=0.6)
3 means Hydroxypropyl-β-cyclodextrin (MS=0.9)
4 means Methyl-γ-cyclodextrin (DS=1.8)
5 means Methyl-α-cyclodextrin (DS=1.8)

### Example 2:

### Preparation of a concentrate solution of a complex of Amical in methyl-β-cyclodextrin (DS=1.8)

100 g of methyl-β-cyclodextrin (DS=1.8) are dissolved in 70 ml of water under while stirring. After addition of 6.0 g of Amical the reaction mixture was stirred for 24 h at room temperature. After filtration a clear, aqueous and concentrate solution with a content of 2.83% (w/w) of Amical was obtained.

### Example 3:

### Preparation of a solid Amical inclusion complex

10 g of methyl-β-cyclodextrin (DS=1.8) were dissolved in 100 ml of water. After the addition of 0.5 g of Amical the solution was shaken for 48 hours. Excess of Amical was filtered off and the remaining solution was freeze-dried. 9.95 g of solid Amical/cyclodextrin complex were obtained. The concentration of Amical in the solid was determined at 3.49%.

### Example 4:

### Determination of Minimum Inhibitory Concentrations (MIC)

The efficacy of Amical in comparison to Amical/methyl-β-cyclodextrin inclusion compound against different microorganism was tested by measuring the Minimum Inhibitory Concentrations (MIC in µg/ml). For the cell cultures Sabouraud media were used. Table 1 demonstrates the higher efficacy of the inclusion compound.

**Table 1**

| Comparison of MIC of Amical in free form and as inclusion compound | | | |
|---|---|---|---|
| microorganism | MIC of Amical | MIC of the inclusion compound (example 2) | MIC of inclusion compound calculated on pure Amical |
| Penicillium citrinum | 3.8 | 8 - 30 | 0.23 - 0.85 |
| Aspergillus niger | 1.2 | 4 - 10 | 0.11 - 0.28 |
| Cladosporium cladosporioides | 1.3 | 8 - 15 | 0.23 - 0.42 |
| Rhizopus oryzae | 7.5- 14 | 15 - 50 | 0.42 - 1.41 |

## Claims

1. Inclusion complex of Amical with a cyclodextrin derivative.

2. Inclusion complex according to claim 1 characterized in that the cyclodextrin derivative is an alkylated or hydroxyalkylated cyclodextrin derivative.

3. Inclusion complex according to claim 2 characterized in that the cyclodextrin derivative is a methylated cyclodextrin derivative.

4. Inclusion complex according to claim 1, 2 or 3 characterized in that the cyclodextrin derivative has a degree of substitution of 0.3 to 2.6.

5. Inclusion complex according to any of claims 1 to 4 characterized in that cyclodextrin derivative and amical are present in a ratio of 1 : 0.05 to 1 : 1 (molar ratio).

6. Process for the production of an inclusion complex according to any of claims 1 to 5, characterized in that Amical is stirred, shacked, or kneaded with a cyclodextrin derivative in an appropriate solvent and the resulting product is dried.

7. Aqueous solution characterized in that it contains besides the inclusion complex according to one or more of claims 1 to 5 a compound selected from the group water, C1 - C4 alcohols, acetone, methyl ethyl ketone, ethyl acetate, methyl acetate, dimethyl sulfoxide, dimethyl formamide and pyridine.

8. Aqueous solution according to claim 7 characterized in that it contains a compound selected from the group anti-foaming agent, corrosion inhibitor, emulsifier, inorganic water-soluble salt and surfactant.

9. Use of an inclusion complex according to one or more of claims 1 to 5 or an aqueous solution according to claim 7 or 8 for the preservation of products.

10. Paints, lacquers, aqueous emulsion and dispersion, metal working fluids, cosmetic products, cleansing agents characterized in that they contain an inclusion complex according to one or more of claims 1 to 5 or an aqueous solution according to claim 7 or 8.
